# EUROPEAN PATENT SPECIFICATION

(11) **EP 3 641 889 B1**
(45) Date of publication and mention of the grant of the patent: **25.02.2026**
(21) Application number: 18740905.7
(22) Date of filing: 20.06.2018
(51) Int. Cl.: A61P 25/28, A61K 31/164, A61K 36/537

(54) **A COMPOSITION FOR TREATMENT OR PREVENTION OF A NEURODEGENERATIVE DISEASE**
ZUSAMMENSETZUNG ZUR BEHANDLUNG ODER VORBEUGUNG EINER NEURODEGENERATIVEN ERKRANKUNG
COMPOSITION POUR LE TRAITEMENT OU LA PRÉVENTION D'UNE MALADIE NEURODÉGÉNÉRATIVE

(30) Priority: 20.06.2017 IT 201700068608
(43) Date of publication of application: 29.04.2020
(73) Proprietor: Neilos S.r.l., 80063 Piano di Sorrento (NA) (IT)
(72) Inventor: DI MAIO, Umberto, 80063 Piano Di Sorrento (NA) (IT)
(74) Representative: Longoni, Alessandra
(86) International application number: PCT/IB2018/054550
(87) International publication number: WO 2018/235016

(56) References cited:
- EP-A1- 2 985 037
- WO-A1-2016/193905
- SKAPER STEPHEN D ET AL: "N-Palmitoylethanolamine and Neuroinflammation: a Novel Therapeutic Strategy of Resolution", MOLECULAR NEUROBIOLOGY, HUMANA PRESS, US, vol. 52, no. 2, 9 June 2015 (2015-06-09), pages 1034 - 1042, XP035532215, ISSN: 0893-7648, [retrieved on 20150609], DOI: 10.1007/S12035-015-9253-8
- DATABASE WPI Week 201748, Derwent World Patents Index; AN 2017-406381, XP002778772
- DATABASE WPI Week 201732, Derwent World Patents Index; AN 2017-20547Q, XP002778773
- DATABASE WPI Week 200955, Derwent World Patents Index; AN 2009-M30302, XP002778774
- YU HUIMIN ET AL: "Neuroprotection against A[beta]25-35-induced apoptosis bySalvia miltiorrhizaextract in SH-SY", NEUROCHEMISTRY INTERNATIONAL, ELSEVIER, AMSTERDAM, NL, vol. 75, 13 June 2014 (2014-06-13), pages 89 - 95, XP029008378, ISSN: 0197-0186, DOI: 10.1016/J.NEUINT.2014.06.001
- DATABASE BIOSIS [online] BIOSCIENCES INFORMATION SERVICE, PHILADELPHIA, PA, US; 15 March 2016 (2016-03-15), PALMA ELEONORA ET AL: "Acetylcholine receptors from human muscle as pharmacological targets for ALS therapy", XP055450537, Database accession no. PREV201600348610
- PROCEEDINGS OF THE NATIONAL ACADEMY OF SCIENCES OF THE UNITED STATES OF AMERICA, vol. 113, no. 11, 15 March 2016 (2016-03-15), pages 3060 - 3065, ISSN: 0027-8424, DOI: 10.1073/PNAS.1600251113
- DATABASE WPI Week 201321, Derwent World Patents Index; AN 2013-B31577, XP002778775
- TIAN ET AL: "Salvianolic acid B, an antioxidant from Salvia miltiorrhiza, prevents 6-hydroxydopamine induced apoptosis in SH-SY5Y cells", INTERNATIONAL JOURNAL OF BIOCHEMISTRY AND CELL BIOLOGY, PERGAMON, GB, vol. 40, no. 3, 1 January 2008 (2008-01-01), pages 409 - 422, XP022486810, ISSN: 1357-2725, DOI: 10.1016/J.BIOCEL.2007.08.005
- JAVEDA SANA ET AL: "Tanshinones and mental diseases: from chemistry to medicine", REVIEWS IN THE NEUROSCIENCES, TEL AVIV, IS, vol. 27, no. 8, 30 November 2016 (2016-11-30), pages 777 - 791, XP009503446, ISSN: 0334-1763, DOI: 10.1515/REVNEURO-2016-0012
- SCUDERI CATERINA ET AL: "Neuroglial Roots of Neurodegenerative Diseases: Therapeutic Potential of Palmitoylethanolamide in Models of Alzheimer's Disease", CNS & NEUROLOGICAL DISORDERS, BENTHAM SCIENCE PUBLISHERS LTD., NL, vol. 12, no. 1, 1 February 2013 (2013-02-01), pages 62 - 69, XP009503447, ISSN: 1871-5273, DOI: 10.2174/1871527311312010011
- ZHANG XIU-ZHEN ET AL: "Salvia miltiorrhiza: A source for anti-Alzheimer's disease drugs", PHARMACEUTICAL BIOLOGY, SWETS AND ZEITLINGER, LISSE, NL, vol. 54, no. 1, 2 January 2016 (2016-01-02), pages 18 - 24, XP009503445, ISSN: 1388-0209, DOI: 10.3109/13880209.2015.1027408

## Description

The present invention is defined in the claims. It relates to a composition for treatment and/or prevention of neurodegenerative diseases comprising at least a vegetable extract, an amide of a fatty acid and a D-amino acid. Neurodegenerative disorders are characterised by the progressive loss of neurons from specific regions of the brain. Neurodegenerative disorders include Parkinson's disease, Huntington's disease and Alzheimer's disease, amyotrophic lateral sclerosis (SLA) and pathologies generally considered less grave, but nonetheless debilitating, such as senile dementia.

In the case of Parkinson's disease and Huntington's disease, the loss of neurons constituting the basal ganglia leads to abnormalities in management of movements; Alzheimer's disease, on the other hand, is characterised by the loss of neurons at the level of the hippocampus and the cortex, which leads to dysfunctions in the memory and cognitive processes; Amyotrophic lateral sclerosis (ALS) is a pathology wherein the muscle weakness derives from the degeneration of spinal, bulbar and cortical motor neurons. Overall, these disorders are quite widespread and represent a significant burden for society. They generally emerge in the advanced stages of life, in neurologically normal individuals, but some cases of development of these pathologies have been observed during childhood. Parkinson's disease occurs in more than 1% of individuals over the age of 65 years, while the prevalence of Alzheimer's disease is higher, corresponding to 10% of the population. Huntington's disease is a hereditary disorder with an autosomal dominant character, the incidence of which is significantly lower than that of other neurodegenerative pathologies, but more than 50% of individuals in the families carrying the gene are affected. ALS is also very rare, but it quickly leads to disability and death.

One of the peculiar characteristics of these pathologies is the selectivity of the pathogenesis process for specific types of neurons. For example, in Parkinson's disease, extensive destruction of dopamine neurons in the substantia nigra occurs, while neurons in the cortex and other areas of the brain are not affected.

Neural damage induced by Alzheimer's disease affects the neurons of the hippocampus and neocortex more severely, and even within the cerebral cortex there is no uniformity observed in the areas of neurons affected. Of even greater interest is the observation that in Huntington's disease the mutant gene responsible for this disorder is expressed throughout the brain and in many other organs, but the pathophysiological changes are observed more greatly at the neostriatum level.

The diversity in the pathogenesis of these neurodegenerative disorders has led experts to propose the mechanism of pathogenesis as a combination of genetic and environmental factors that affect the intrinsic physiological characteristics of populations of neurons. These factors may include susceptibility to excitotoxicity-induced damage and the production of toxic free radicals as by-products of cell metabolism. For a long time, it has been suspected that genetic predisposition may have a key role in the etiopathogenesis of neurodegenerative disorders and, until now, several possible mechanisms have been found.

For example, Huntington's disease is transmitted in a hereditary manner and the genetic defect responsible has been identified. In Parkinson's disease, however, mutations of four different proteins can lead to genetically-determined forms of the pathology: a-synuclein, parkin, UCHL1 and DJ-1.

In Alzheimer's disease, mutations in the genes encoding the precursor of the amyloid protein (APP) and of proteins known as presenilins, probably involved in the modification of the APP, lead to hereditary forms of this pathology. A genetic origin of these diseases is, however, rather rare.

Environmental factors are certainly more important etiological agents: infectious agents, toxins and brain damage can contribute to the process of pathogenesis. These factors are more relevant in Parkinson's disease, which can be caused by encephalitis or toxins such as "synthetic heroin", MPTP (N-methyl-4-phenyl-1 ,2,3,6-tetrahydropyridine) or rotenone, which are able to damage dopamine neurons and lead to a condition very similar to Parkinson's disease. Traumatic brain damage can lead to the onset of Alzheimer's disease.

Excitotoxicity is a phenomenon resulting from the excessive concentration of glutamate in the brain. Although glutamate is essential for normal brain function, its excessive concentration can lead to neuronal death induced by excitotoxicity. The destructive effects of glutamate are mediated by glutamate receptors, particularly those of NMDA (N-methyl-D-Aspartate): the activation of this channel receptor by the glutamate leads to the ingress of Ca²⁺ ions which, at high concentrations, are able to induce a series of biological processes with destructive effects on neuronal cells.

Oxidative stress also plays a key role in the pathogenesis process. Excessive production of reactive compounds such as hydrogen peroxide and reactive oxygen species can lead to DNA damage, membrane lipid peroxidation and neuronal death. Alzheimer's disease (AD) produces a progressive weakening of cognitive abilities. Short-term memory dysfunction is generally the first clinical sign, while long-term memory recall is relatively well preserved during the progression of the pathology. In the most advanced phases of this pathology, several other cognitive abilities decay, such as the ability to calculate, to exert visual-spatial abilities and the ability to use objects and tools (ideomotor apraxia). The level of excitation and the patient's degree of alertness are not damaged until the most advanced stages of the pathology. Death, most often resulting from complications linked to immobility such as pneumonia or pulmonary embolism, generally occurs 6-12 years after the onset of the pathology.

The diagnosis of Alzheimer's disease is based on careful clinical
evaluation of the patient and appropriate laboratory tests to ensure that other AD-like disorders are counted out.

Alzheimer's disease is characterised by a marked atrophy of the cerebral cortex and the loss of cortical and subcortical neurons. The pathological characteristics of AD are senile plaques, which are spherical accumulations of the β-amyloid protein accompanied by degenerative neuronal processes and the formation of neurofibrillary tangles. The amount of senile plaques and neurofibrillary tangles is directly proportional to the cognitive deficits associated with this pathology. These signs of the pathology are prevalent in the hippocampus and in the associative regions of the cortex, while areas such as the visual cortex and motor cortex are relatively spared.

Neurochemical disorders arising in AD have been the subject of intensive study. Direct analysis of the content of neurotransmitters in the cerebral cortex reveals a reduction in the concentration thereof in parallel with neuronal loss: in particular, there is a marked and abnormal reduction in the acetylcholine content. The anatomical bases of the cholinergic deficit are atrophy and degeneration of the cholinergic neurons, in particular in the nucleus basalis, where the cholinergic neurons of the entire cortex are innervated. The selective deficit of acetylcholine in AD, as well as the observation of dementia-like condition induced by cholinergic antagonists, led to the "cholinergic hypothesis", an explanation for the process of pathogenesis of Alzheimer's disease.

The presence of β-amyloid aggregates is a particular characteristic of AD. Until recently, it was not entirely clear whether this was a process that could cause the pathology or whether it was an effect thereof, i.e. a consequence of neuronal death. The main pharmacological approach to the treatment of Alzheimer's disease is to try to restore the cholinergic function of the brain. An initial approach was to administer acetylcholine precursors, such as choline chloride and phosphatidylcholine. Although these supplements are generally well tolerated, no significant evidence of the effectiveness of these compounds is apparent from randomised clinical trials. A more promising strategy is represented by inhibition of acetylcholinesterase (AChE), a catabolic enzyme of acetylcholine.

Physostigmine is moderately effective in improving memory in animal learning models: the short half-life and the side effects resulting from cholinergic activation severely limit its use in humans.

Tacrine showed a slight memory enhancing effect when used in combination with choline. Strong side effects limit its use: abdominal cramps, anorexia, nausea, vomiting and diarrhoea, observed in more than a third of patients treated with this drug, and the increase in serum transaminases, observed in more than 50% of patients treated.

Donepezil is a selective inhibitor of AChE in the central nervous system, with minimal effects on peripheral tissues. The effectiveness thereof in treating patients with Alzheimer's disease is very limited.

Rivastigmine and galantamine have similar effects in reducing the symptoms of Alzheimer's disease. The side effects are minor compared to those of tacrine, but they are relevant and consist of nausea, diarrhoea, vomiting and insomnia.

Other pharmacological treatments include the use of glutamate NMDA receptor antagonists, such as memantine. It is not known whether the mechanism of this drug can act on the cause of the pathology. Side effects of memantine include headaches and dizziness.

There is therefore a need to provide treatment for this pathology, which is effective for both treatment and prevention, which is easy to use and which is basically free of the side effects of the therapies that are currently used.

Senile dementia is a progressive neurodegenerative disease of the brain, which over time leads the affected person to have a decrease in the faculties of language, memory and logic.

Historically, the term dementia referred to an irreversible, acquired and global deterioration of mental functions. The disorder is progressive and is believed to be due to brain damage.

Dementia can be classified as follows:
- Dementia due to cerebrovascular pathologies. It may result from disorders of the cerebral and peripheral vessels. Neuropsychiatric symptoms may be caused by hypertensive encephalopathy, by embolism or cerebral haemorrhage, by thrombosis and ischemia. Indeed, cerebral ischemia is the main vascular cause of dementia. The repetition of such events leads to the onset of multi-infarct dementia.
- Dementia due to primary degenerative disorders. Primary degenerative disorders such as Alzheimer's disease and some other presenile dementias.

Senile dementia is a type of dementia that occurs in old age, usually after the age of 65, and is diagnosed in the absence of other possible causes of this disorder.

In the context of the present invention, it is possible to define senile dementia as the progressive loss of cognitive and perceptive faculties that arises in old age, characterised by the absence of a known aetiology". (C. G. Gottfries, "Alzheimer's disease and senile dementia: Biochemical characteristics and aspects of treatment" Psychopharmacology (Berl)., vol. 86, pp. 245-252, 1985.)

In reality, there is not just one type of senile dementia: dementias are part of an extremely heterogeneous group, which includes more or less reversible forms of this disorder.

As in the case of Alzheimer's disease, the morphological changes of senile dementia are observed mainly in the neocortex, especially in the temporal, parietal and occipital area, as well as in the grey matter. There is no epidemiological data relating specifically to senile dementia.

However, some interesting conclusions can be drawn from the numerous statistical studies on the broader category of dementias.

According to these studies, dated 2010, there are around 36 million dementia sufferers worldwide, divided as follows:
3% are between 65 and 74 years old, 19% are between 75 and 84 years old and more than half are 85 and above.

6.8 million live in the United States. More than half of these people with dementia are over 80 years old.

750,000-800,000 live in the United Kingdom.

50-70% suffer from Alzheimer's disease, 25% from vascular dementia, 15% from Lewy body dementia and the remaining percentage is affected by other existing forms of dementia.

The main pharmacological treatments for dementia are the following:
Neuroleptic agents (typical or atypical antipsychotics);
Antidepressant drugs;
Anti-dementia drugs (acetylcholinesterase inhibitors; nootropic agents);
Sedative/hypnotic;
Anticonvulsants.

However, these drugs have significant side effects and are not tolerated by all patients.

There is still a need for treatment that makes it possible to prevent or treat neurodegenerative pathologies and which is effective, basically free of side effects, and tolerable for patients, including the elderly, for prolonged treatment.

As a solution to said necessity, after long and extensive research and development, the inventors have developed a composition for use in a method of treatment, preventive and/or curative, of neurodegenerative diseases according to the appended claims.

The present invention relates to a composition comprising an effective amount of a mixture which comprises or, alternatively, consists of:
a) palmitoylethanolamide,
b) extract of *Salvia miltiorrhiza* with a titre of tanshinones of 10% by weight/total weight of the dry extract and,
c) D-aspartate.

The present invention relates to a composition comprising or, alternatively, consists of an effective amount of a mixture which comprises or, alternatively, consists of:
a) palmitoylethanolamide,
b) extract of *Salvia miltiorrhiza* with a titre of tanshinones of 10% by weight/total weight of the dry extract and,
c) D-aspartate,
for use in a preventive and/or curative treatment method of cognitive deficits induced by neurodegenerative pathologies.

An object of the present invention is a pharmaceutical composition or a food supplement comprising, or alternatively consisting of, an effective amount of a mixture which comprises or, alternatively, consists of:
a) palmitoylethanolamide,
b) extract of *Salvia miltiorrhiza* with a titre of tanshinones of 10% by weight/total weight of the dry extract and,
c) D-aspartate,
and, optionally, at least one inert ingredient, additive or excipient acceptable for pharmaceutical, nutraceutical or food use.

An object of the present invention is a preventive and/or curative treatment method of cognitive deficits induced by neurodegenerative pathologies, which includes administering a composition comprising an effective amount of a mixture that comprises or alternatively consists of:
a) palmitoylethanolamide,
b) extract of *Salvia miltiorrhiza* with a titre of tanshinones of 10% by weight/total weight of the dry extract and,
c) D-aspartate,
and, optionally, at least one inert ingredient, additive or excipient acceptable for pharmaceutical, nutraceutical or food use for a subject in need.

Preferred embodiments of the present invention will become apparent from the detailed description that follows and are indicated in the appended claims.

### Figures:

Figure 1 : Y-maze test;
Figure 2: Object recognition test;
Figure 3: experimental model;
Figure 4: result of the object recognition test;
Figure 5: results of Y maze test;
Figure 6: coordination of the motor activity in the Y-maze test;
Figure 7: ELISA assay.

Within the scope of the present invention, "treatment" or "treatment method" of diseases means a therapy aimed at restoring a subject's conditions of health, maintaining the existing conditions and/or preventing or slowing any worsening of said conditions of health.

Within the scope of the present invention, "prevention" of pathologies means a therapy aimed at preventing the onset of a pathology of this type in a subject, also, but not only, as a complication or effect of a pathological condition or pre-existing disorder.

Unless specified otherwise, within the scope of the present invention the percentages and amounts of a component in a mixture are intended to refer to the weight of that component relative to the total weight of the mixture.

In the context of the present invention the term "composition(s)" can, without limitation, relate to a pharmaceutical composition, , or a composition for a food supplement, or a food composition.

In an aspect, the present invention provides a composition comprising, or alternatively consisting of an effective amount of a mixture that comprises or alternatively consists of:
a) palmitoylethanolamide,
b) extract of *Salvia miltiorrhiza* with a titre of tanshinones of 10% by weight/total weight of the dry extract and,
c) D-aspartate, for use in a preventive and/or curative treatment method of cognitive deficits induced by neurodegenerative pathologies.

The invention is based on the synergistic action of the aforesaid active ingredients a), b) and c).

Palmitoylethanolamide, also known as PEA, N-Palmitoylethanolamine, N-(2-hydroxyethyl)hexadecanamide or Palmidrol (CAS Number 544-31-0), is an amide deriving from palmitic acid and ethanolamine. It belongs to the N-acylethanolamine family, which also includes anandamide, the endogenous ligand of the cannabinoid and oleoylethanolamide receptors. PEA was identified for the first time in the 1950s, as an anti-inflammatory compound isolated in egg yolk.

Preclinical and clinical studies suggest that the PEA might potentially be useful in treatment of various pathologies, such as eczema and various types of pain and, at the same time which might be free of undesired effects in humans.

Palmitoylethanolamide has shown its effectiveness in a variety of pain models, among which hyperalgesia induced by carrageenans and prostaglandins, in the persistent pain test induced by formalin, in visceral hyperalgesia produced by NGF instillation (nerve growth factor) of the bladder and in the neuropathic pain model induced by sciatic nerve ligation. However, it has not shown itself to be particularly effective in heat hyperalgesia.

There exist various mechanisms which explain the pharmacological action of PEA. This substance can, in fact, act in a direct or indirect manner on various receptors such as PPAR-a, GPR55, receptors of the endocannabinoid system and TRPV1 channel receptor.

Only few research studies are at present available relating to the pharmacokinetics of palmitoylethanolamide. The oral bio-availability (Fₒₛ) and apparent volume of distribution (V_{d}) have in fact not yet been determined. The tissue distribution of palmitoylethanolamide has also been researched. In a study carried out by Artamonov et al., the distribution of N-[9,10-3H] PEA was measured in Wistar rats of 150-200 g, 20 minutes following the administering (doses of about 100mCi, 3.3x10 ⁻⁵ mol/100 g of body weight, corresponding to approximately 100 mg kg ⁻¹). The authors of this study observed that about 0.95% of the PEA administered was distributed to the brain, with a heterogeneous distribution in the various parts of the brain. This is surprising for a lipophilic compound and suggests a preferential retention in the hypothalamus. An explanation of this phenomenon might be a portion of the hypothalamus provided with particular bonding affinity for PEA (M. Artamonov, O. Zhukov, and I. Shuba, "Incorporation of labelled N-acylethanolamine (NAE) into rat brain regions in vivo and adaptive properties of saturated NAE under x-ray irradiation", Ukr Biokhim Zh (1999) 2005; 77(6):51-62.).

Palmitoylethanolamide can be useful in the treatment of diseases associated with or resulting from neurodegeneration, hyperalgesia and inflammation, as well as brain damage resulting from ischemia. PEA also has a protective effect on neurological disorders induced by cerebral ischemia and traumatic brain damage. In these conditions, in fact, an increase in tissue levels of PEA in the central nervous system is often observed, since this molecule has the function of protecting neurons from damage induced by ischemia and brain trauma.

*Salvia miltiorrhiza,* the botanical name of the plant known as red sage or Chinese sage, is widely used in traditional Chinese medicine for the treatment of cerebrovascular and cardiovascular disorders, as well as for inflammatory diseases. Tanshinones, diterpenic compounds with a structure similar to that of abietane, are the main chemical compounds contained in extract of *Salvia miltiorrhiza.* The main bioactive compounds are tanshinone I (TNI), tanshinone IIA (TNIIA) and cryptotanshinone (CPT). These compounds have attracted attention because of their different pharmacological effects, which include the anti-inflammatory and antitumour effect and cerebrovascular protection activities. Tanshinone IIA has potential effects against diabetes, neurodegenerative pathologies and cardiac hypertrophy. In addition to the anti-inflammatory and antitumour effects thereof, tanshinone I has the ability to improve memory and learning ability and lead to the improvement of memory disorders.

Several studies have been carried out to evaluate the activity of *Salvia miltiorrhiza* and tanshinones in the treatment of cardio- and cerebrovascular pathologies, such as myocardial infarction, atherosclerosis, hyperlipemia, hypertension and stroke. -

Tanshinone IIA and tanshinone IIA sodium sulphonate have been shown to be effective in reducing the extent of myocardial infarction and improving cardiac function by activating phosphatidylinositol 3-kinase (PI3K). Other pharmacological effects consist in inhibiting platelet aggregation, inhibiting the formation of atherosclerotic lesions and hyperlipemia, reducing the oxidation of lipoproteins and the accumulation of macrophages at the level of the vascular tunicae.

Other pharmacological activities of tanshinones consist in the inhibition of tumour cell proliferation. The antitumour action mainly derives from the cytotoxicity of TNIIA, TNI and CPT, with the interruption of the cell cycle and apoptosis. The mechanism might consist of the up-regulation of pro-apoptotic proteins. Tanshinone IIA also showed important anti-inflammatory activity, inhibiting the expression of inflammatory mediators such as IL-1β, IL-6 and tumour necrosis factor α (TNF-α). Further, TNIIA can inhibit the activation of the lipopolysaccharide-induced NF-kB, by inhibiting the NIK/IKKα pathways (NF-KB-inducing kinase/ IkappaB alpha kinase), ERK-1/2, p38, and JNK (c-Jun N-terminal kinase). The anti-inflammatory effect might also derive in part from the activation of the factor associated with the NRT receptor (TRAF) 2/3/6 and the inhibition of the signalling pathway of the toll-like receptor (TLR).

Tanshinones are known as natural antioxidants. The mechanism consists of the formation of a quinone adduct of the lipid radical to form a stabilized radical.

Typically, extracts of *Salvia mitiorrhiza* are titrated into tanshinone IIA. The extracts of *Salvia miltiorrhiza* in the composition according to the invention have an IIA tanshinone content of 10% by weight/total weight of the dry extract.

For the purpose of the present invention, D-amino acid means the D-enantiomer of an alpha- or beta-amino acid, of natural or synthetic origin, typically containing less than 0% by weight, preferably less than 5% or 1% or 0.5% or 0.1% by weight of the L-isomer. It is understood that in the composition of the invention the D-amino acid may be, alternatively, in free form, such as salt, or in the form of a pharmacologically active derivative, pro-drug, metabolite, similar or other forms known to the expert in the field of pharmaceutical, food and nutraceutical formulations.

In the composition of the present invention, the D-amino acid is D-aspartate..

It is believed that cognitive processes depend on changes in synaptic efficacy in key brain regions, in particular in the hippocampus. Among the different forms of synaptic plasticity, long term potentiation (LTP) dependent on NMDA (N-methyl-D-aspartate) receptors of glutamate was proposed as the most likely mechanism behind the synaptic plasticity of spatial learning and the memory. Antagonists of this receptor inhibit long-term potentiation and damage mnemonic abilities in rats. Long-term potentiation is improved in rats with over-expression of the NR2B subunit of this receptor. Even in elderly rats, inducing the expression of this subunit leads to improvements in cognitive faculties.

Although in the past it was believed that D-amino acids were used exclusively by bacteria, recent experimental evidence has demonstrated that they are involved in the neurocognitive processes of mammals. In particular, D-serine, acting as an endogenous co-agonist of the glycine binding site of the NMDAR receptor, has the ability to restore the decline in cognitive faculties induced by the malfunctioning of NMDAR receptors in old age.

Another important D-amino acid found in mammal brain tissue is D-aspartate.

It is a natural amino acid found in all animal phyla, including humans. It was discovered in the brain of the mollusc Octopus vulgaris following the search for a ligand for the enzyme D-aspartate oxidase. D-aspartate is found mainly in the endocrine and nervous systems, where it is involved in important physiological functions. In the central nervous system, D-aspartate is involved in neuromodulation of cell-cell signalling and is able to improve learning and memory in rats, improve synaptic plasticity resulting from Long Term Potentiation and induce growth of neuronal dendrites in rats.

Experimental evidence shows that this amino acid is present in high concentrations in the brain during the stage of development, while in the post-natal phase concentrations tend to progressively decrease. This phenomenon is very likely to be associated with the expression of D-aspartate oxidase, an enzyme responsible for the degradation of this amino acid. In accordance with in vitro binding affinity studies, which indicate the ability of D-aspartate to bind the NMDA glutamate receptor, it has been demonstrated that when the endogenous content of this amino acid exceeds endogenous levels there is an increase in long-term potentiation in the hippocampus and a suppression of long-term depression in the striatum. The beneficial effects of D-aspartate on the central nervous system have led to investigating the effects of this amino acid in cognitive behaviour in numerous experiments on mice.

The main pharmacological characteristic of D-aspartate to act as an endogenous agonist of NMDA glutamate receptors is in line with its role in modulating synaptic plasticity in CA1 synapses of the hippocampus. It has recently been demonstrated that the non-physiological increase in D-aspartate in the mouse hippocampus, obtained by removal of the D-aspartate oxidase encoding gene or by treatment with D-aspartate for one month, can increase the NMDAR-dependent long-term potentiation. In a preclinical study it was shown that minute changes in D-aspartate hippocampal levels above physiological levels in the nmol/g range of tissue are capable of regulating the synaptic plasticity of CA1 synapses. It has been observed that a doubling of the D-aspartate hippocampal concentration in the hippocampus, obtained by administration of this amino acid for 3 months, is sufficient to induce a higher NMDAR-dependent LTP. Suspension of treatment for 3 weeks is sufficient to restore physiological levels of D-aspartate and, consequently, the amplitude of the LTP; however subsequent treatment of mice with D-aspartate for 1 month is able to bring the levels back to those prior to interruption of treatment. This suggests that phase changes in the concentration of D-aspartate, induced by oral administration of this amino acid, are responsible for modulation of synaptic plasticity in mice.

D-aspartate can be used either in the form of D-aspartic acid, or in the form of sodium salt (D-aspartate), or in the anhydrous or hydrated form.

Preferably, in the composition according to the present invention, a) is present in an amount comprised between 1% and 50%, more preferably between 2% and 30%, b) is present in an amount comprised between 0.1 % and 40%, more preferably between 1 % and 20% and/or c) is present in an amount comprised between 10% and 95%, more preferably between 30% and 90% by weight/total weight of the composition.

In a preferred embodiment, the composition according to the present invention is for use in a treatment, curative and/or preventive treatment method, of cognitive deficits induced by neurodegenerative pathologies (pathologies associated with neurodegenerative disease).

In a preferred embodiment, the composition according to the present invention is for use in a treatment, curative and/or preventive treatment method, of cognitive deficits induced by neurodegenerative pathologies such as Parkinson's disease, Huntington's disease, Alzheimer's disease, amyotrophic lateral sclerosis and senile dementia.

The composition according to the present invention can be used in a treatment, curative and/or preventive method of senile dementia.

The composition according to the present invention can be for use in human subjects or veterinary use, for example, but without limitation, in pets such as dogs, cats or other mammals. The composition according to the present invention is preferably for use in humans.

In an embodiment, the administering of the composition to the subject takes place orally, for example in the form of a pill or tablet, which may also be coated, capsule, solution, suspension, syrup, foodstuff containing probiotic bacteria or in any other form known to the person skilled in the art.

It is understood that said administration of at least a), b) and, c) according to the invention can take place simultaneously, for example in a single formulation, or in a rapid sequence, for example through two or more formulations taken by the subject in any order, in a close sequence over time (e.g. within 1 to 30 minutes) in two distinct compositions.

The composition for use according to the present invention can comprise, in addition to a), b) and c), at least one inert ingredient, such as at least one excipient among the ones commonly used and known to the person skilled in the art.

"Inert ingredient" means any substance, or combination of substances, auxiliary to the production of a pharmaceutical, dietary or nutraceutical form, which is to be found in the finished product and is not the active ingredient, although it can modify the stability, release or other characteristics thereof.

Non-limiting examples of such ingredients, as known to the person skilled in the art of formulations in the pharmaceutical, nutraceutical or food sectors, include excipients such as diluents, absorbents, adsorbents, lubricants, glidants, colourants, surfactants, antioxidants, sweeteners, flavourings, bindings, disintegrants, plasticisers, viscosity enhancers, emulsifiers, humectants, wetting agents, preservatives and chelating agent and the like.

In one embodiment, the composition for use according to the present invention comprises, in addition to active ingredients a), b) and c), at least one further active ingredient of natural or synthetic origin. Non-limiting examples of said active ingredients are the B-group vitamins, in particular thiamine; antioxidants, such as vitamin C and vitamin E; neurotrophic and/or nootropic agents, such as citicoline, acetyl-L-carnitine, phosphatidylserine, phosphatidylcholine and plant extracts of the genera *Panax* and *Bacopa.*

In an embodiment, the present invention provides a pharmaceutical composition or a food supplement comprising, or alternatively, consisting of an effective amount of a mixture which comprises or, alternatively, consists of:
a) palmitoylethanolamide,
b) extract of *Salvia miltiorrhiza* with a titre of tanshinones of 10% by weight/total weight of the dry extract and,
c) D-aspartate and, optionally, at least one inert ingredient, additive or excipient acceptable for pharmaceutical, nutraceutical or food use.

In a preferred embodiment, the present invention provides al composition as such, a pharmaceutical composition or a food supplement comprising or, alternatively, consisting of an effective amount of a mixture which comprises or, alternatively, consists of:
a) palmitoylethanolamide,
b) extract of *Salvia miltiorrhiza* with a titre of tanshinones of 10% by weight/total weight of the dry extract
c) D-aspartate and, optionally, at least one inert ingredient, additive or excipient acceptable for pharmaceutical, nutraceutical or food use.

In a further preferred embodiment, the present invention provides a composition as such, a pharmaceutical composition or afood supplement comprising or, alternatively, consisting of an effective amount of a mixture which comprises or, alternatively, consists of:
a) palmitoylethanolamide,
b) extract of *Salvia miltiorrhiza* with a titre of tanshinones of 10% by weight/total weight of the dry extract
c) D-aspartate,
d) a vitamin from the B group, preferably vitamin B1
and, optionally, at least one inert ingredient, additive or excipient acceptable for pharmaceutical, nutraceutical or food use.

In the composition according to the present invention, preferably a) is present in an amount by weight comprised between 1 % and 50% of the total weight of the composition, preferably between 2% and 30%, b) is present in an amount by weight comprised between 0.1% and 40% of the total weight of the composition, preferably between 1% and 20% and/or c) is present in an amount by weight comprised between 10% and 95%, preferably between 30% and 90% by weight/total weight of the composition.

In the context of the present invention, the term "medical device" is used with the meaning according to Italian Legislative Decree no. 46 of 24 February 1997, i.e. it indicates a substance or another product, whether used alone or in combination, intended by the manufacturer to be used for human beings for the purpose of diagnosis, prevention, monitoring, treatment or alleviation of a disease, and which does not exert its principal action in or on the human body for which it is intended, by pharmacological or immunological means or metabolic means, but which may be assisted in its function by such means.

The pharmaceutical composition or food supplement of the present invention can be solid, semi-solid, for example a suspension or gel, and can be in any form known to the person skilled in the sector of food, pharmaceutical or nutraceutical formulations, such as, by way of non-limiting example, in the form of a capsule, tablet, or powder that is at least partially dissolvable in the mouth or water soluble, granules, pellets or microparticles, optionally contained in a sachet or in a capsule (mini-tablet), liquid or semisolid preparation, gel, suspension, solution, two-phase liquid system and equivalent forms.

In the pharmaceutical composition or food supplement according to the present invention, preferably a) is present in an amount by weight comprised between 1% and 50% of the total weight of the composition, preferably between 2% and 30% b) is present in an amount by weight comprised between 0.1% and 40% of the total weight of the composition, preferably between 1% and 20% and/or c) is present in an amount by weight comprised between 10% and 95%, preferably between 30% and 90% by weight/total weight of the composition.

The following experimental part provides examples of practical embodiments of the invention, without limiting the scope thereof.

The following experimental part demonstrates that compositions (or formulations) according to the invention are effective for use in a method for treating pathologies associated with neurodegenerative pathologies such as, for example, Parkinson's disease, Huntington's disease, Alzheimer's disease, amyotrophic lateral sclerosis (ALS) and senile dementia. In particular, behavioural tests (Y-maze test and Object recognition test) show that mice treated with compositions (or formulations) according to the invention show an improvement in cognitive functions compared to mice treated with the single ingredients or combinations of individual ingredients other than the composition (or formulations) according to the invention. Further, in none of the experimental conditions an alteration of motor coordination has been detected (Y-maze test); therefore, the compositions (or formulations) of the invention that have been tested are free of side effects, at least in relation to the alteration of motor coordination.

### DESCRIPTION OF THE FORMULA or FORMULATION

In the present invention the synergistic action takes place between palmitoylethanolamide, the extract of the *Salvia miltiorrhiza* plant and D-aspartate.

Synergy is most pronounced when the palmitoylethanolamide is present in an amount (dose) by weight ranging from 10 mg to 5000 mg, preferably 40 mg to 2500 mg, and even more preferably 80 mg to 1000 mg; an extract of a plant of the genus *Salvia,* preferably *Salvia miltiorrhiza,* is present in an amount (dose) ranging from 10 mg to 10000 mg preferably from 2 mg to 800 mg, even more preferably from 50 mg to 400 mg; and D-aspartate is present in an amount (dose) ranging from 10 mg to 10000 mg, preferably from 100 mg to 8000 mg, even more preferably from 500 mg to 6000 mg. Doses refer to the mixture of the active ingredients.

By the terms formula or formulation, in the context of the present invention, are meant the pharmaceutical composition or food supplement of the present invention administered to the subject in need, i.e. including both the active ingredients and any other ingredient.

Some non-limiting examples of embodiments of the present invention are reported below:

### EXAMPLE 1

| Active ingredient | Daily dose |
|---|---|
| D-Aspartate | 4000 mg |
| Palmitoylethanolamide | 400 mg |
| *Salvia miltiorrhiza,* dry extract (d.e.) | 200 mg |

### EXAMPLE 2

| Active ingredient | Daily dose |
|---|---|
| D-Aspartate | 2000 mg |
| Palmitoylethanolamide | 400 mg |
| Dry extract of *Salvia miltiorrhiza* | 200 mg |

### EXAMPLE 3

| Active ingredient | Daily dose |
|---|---|
| D-Aspartate | 2000 mg |
| Palmitoylethanolamide | 200 mg |
| Dry extract of *Salvia miltiorrhiza* | 200 mg |

### EXAMPLE 4

| Active ingredient | Daily dose |
|---|---|
| D-Aspartate | 2000 mg |
| Palmitoylethanolamide | 200 mg |
| Dry extract of *Salvia miltiorrhiza* | 100 mg |

### EXAMPLE 5

| Active ingredient | Daily dose |
|---|---|
| D-Aspartate | 1000 mg |
| Palmitoylethanolamide | 200 mg |
| Dry extract of *Salvia miltiorrhiza* | 100 mg |

### EXPERIMENTAL PART

To determine the synergistic effect of the components of the formulation, the following are administered orally (P.O.), individually and in combination: palmitoylethanolamide, an extract of *Salvia miltiorrhiza* (whit a known titre of tanshinones, which have been shown to have beneficial effects on cerebral microcirculation and the state of neuroinflammation); and D-aspartate for a time-course of I, 4 weeks using a mouse model of Alzheimer's disease (AD) induced by intracerebral-ventricular (i.c.v.) administration of the fibrilled form of peptide 31-42 (peptide involved in pathogenesis of AD). For each time-course the behavioural response of the animals (in vivo experiments) in terms of memory and motor coordination will be evaluated using behavioural tests such as Y-maze and Object recognition. Lastly, for each time-course on the cortex and/or hippocampus lysates (in vitro experiments) of each animal (about 10 animals per experimental condition are used) molecular biology assays (Western Blot and ELISA assay) will be performed in order to evaluate the modulation of typical neuroinflammation markers such as: TNF-α, IL-1β and IL-6.

### Materials

(a) Peptide β1-42 (referred to as "Beta"): Amyloid β peptide 1-42; Tocris Milan Italy, CAS 107761-42-2.
(b) Palmitoylethanolamide (referred to as "PEA"): Fraupharma, Ref. PEAM00008NT17).
(c) *Salvia miltiorrhiza* 10% (referred to as "Salvia"): *Salvia miltiorrhiza* with a known titre of tanshinones; 10%; Arda Natura SRL, Ref. N21102L0).
(d) D-aspartate (referred to as "Aspartate"): D-aspartic acid; Barenz Service S.p.A, Ref. 20170331).
   - Composition I according to the invention (*salvia miltiorrhiza* 10% + palmitoylethanolamide; referred to as "Salvia+PEA") and
   - Composition II according to the invention (*salvia miltiorrhiza* 10% + palmitoylethanolamide + D-aspartate; referred to as "Formula")
      Animals for *in vivo* experiments: CD1 strain mice, weight 20-25g (Charles River Italy).
   - Elisa Mouse Cytokine Array Kit (ELISA Kit) and relative quantification kits from R&D System (Ref. P157472).

### Methods

Using an induced Alzheimer's disease (AD) mouse model of the fibrillated form of (a) β1-42 (Beta) peptide intracerebroventricularly administered to mice (ICVs) at the dose of 3 g/3 L, substances (b)-(d) were administered to the animals orally individually and/or in combination for a 4-week time-course (1 week for settling and 3 weeks of testing). In particular, substances (b)-(d), singly and/or in combination, have been administered at the following doses:
(b) PEA equivalent to a dose of 400 mg in a human being;
(c) *Salvia miltiorrhiza* 10% equivalent to a dose of 200 mg in a human being;
(d) D-aspartate equivalent to a dose of 2000 mg in a human being.

The substances were administered in combination, such as:
- composition I according to the invention (*salvia miltiorrhiza* 10% + palmitoylethanolamide; referred to as "Salvia+PEA") and
- composition II according to the invention (*salvia miltiorrhiza* 10% + palmitoylethanolamide + D-aspartate; referred to as "Formula").

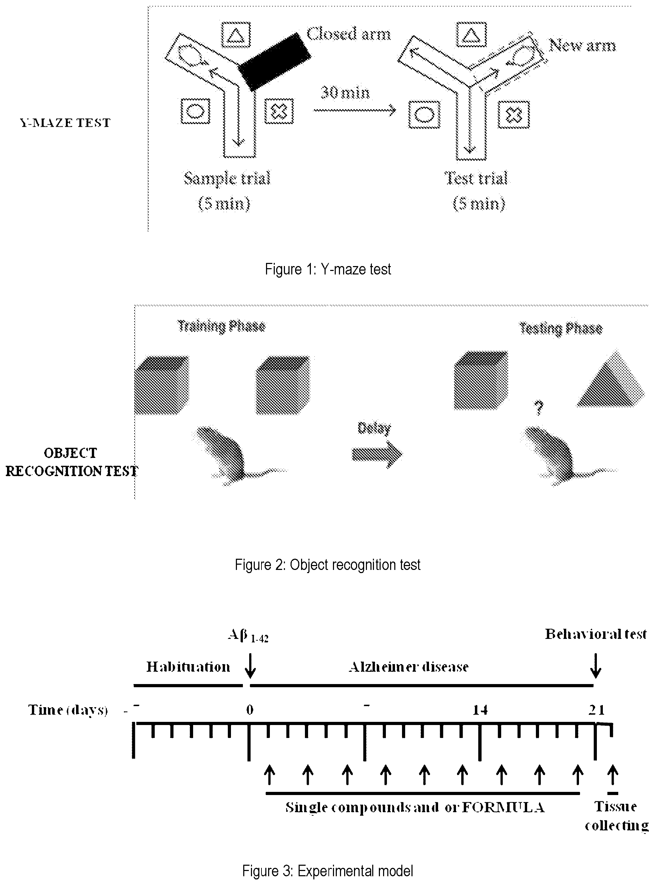

To assess the behavioural memory response of the animals, *in vivo* tests such as Y-maze (Figure 1) and Object recognition (Figure 2) (behavioural tests) were carried out.

Further, molecular biology assays (ELISA assays) were performed on the cortex and hippocampus lysates of each animal to assess the modulation of the major inflammatory cytokines (ex vivo experiments).

Figure 3 shows the schematic representation of the experimental model for the administration of (a) β1-42 peptide (Alzheimer's disease) and the substances (b)-(d), singly or in combination ("Salvia+PEA" and "Formula"), and the performing of *in vivo* and *ex vivo* tests. Behavioural tests (*in vivo* tests) were performed on day 21, followed on day 22 by organ removal for subsequent biochemical investigations (*ex vivo* tests).

### Statistics

All results were expressed as mean ± standard error of the mean (S.E.M.) and analysed by one-way analysis of variance (one-way ANOVA) followed by Bonferroni's test for multiple comparisons. Statistical analysis was carried out using GraphPad software version 4.0. Values below P<0.05 were considered significant.

### Y-maze test

Y-maze is a behavioural test to measure the willingness of rodents to explore new environments. Healthy rodents generally prefer to investigate a new arm of the maze rather than return to one that has previously been visited. This test is then used to evaluate the effects of new compounds/compositions on mouse cognitive activity.

The Y-maze used in the present invention consists of three arms 40 cm long and separated from each other by an angle of 120°. Each naive animal was placed in the terminal portion of one of the arms of the Y-maze. The animal was allowed to move freely in the apparatus for 5 minutes. The sequence of entries
into the arms made by the animal, including entries into the arms already explored, was visually recorded. The total number of possible animal entries in all three arms in consecutive trials (referred to as alternation) was therefore the total number of entries minus 2 and this percentage was calculated as (actual alternations/total alternation) x 100.

### Object recognition test

In the Object recognition test ihe animal was subjected to two different sessions (one for training and the other for testing). During the training session, two different objects (A and B) were placed in the test area. Each animal was able to explore both objects for 5 minutes. The time spent on exploration was recorded by an operator. In the next session (testing), one of the two objects was replaced by a new one (C). Again, in this case the animal was able to explore the objects for 5 minutes and the time spent exploring was recorded. The preference for the new object was considered as a preservation of the memory regarding the familiar object.

### Solubility tests

Prior to the experiments described above, solubility tests were carried out on the test compounds (Peptide β1-42, *Salvia miltiorrhiza,* Palmitoylethanolamide, D-aspartate). The results of the solubility tests are schematically shown in Table 1.

**Table 1**

| SUBSTANCE | SOLUBILITY | RESULT |
|---|---|---|
| Peptide β1-42 | 500 µL of Saline | POSITIVE |
| *Salvia miltiorrhiza* | 2700 µL of DMSO Saline (1:3) | POSITIVE |
| Palmitoylethanolamide | 900 µL of Tween 80 + 1800 µL of Peg 460 | POSITIVE |
| D-aspartate | 2700 µL of H₂O | POSITIVE |

### Results

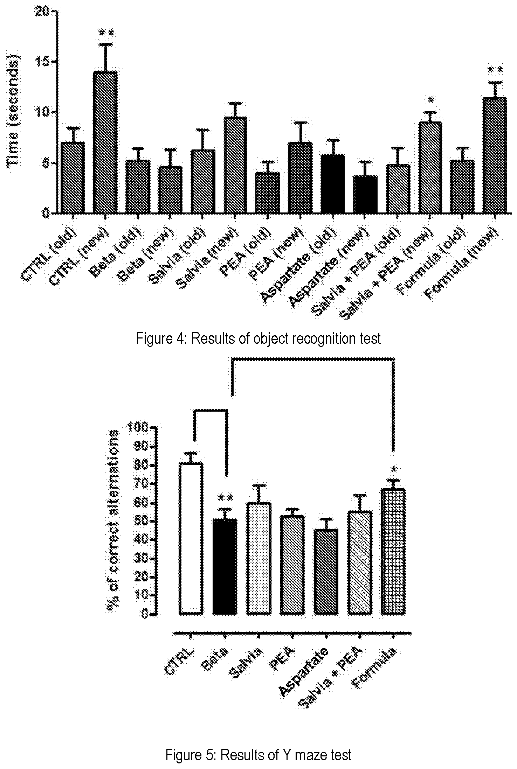

Figure 4 shows the results of the Object recognition test at 3 weeks (1 week of settling + 3 weeks of testing) in the untreated experimental group (control group, CRTL) and in the experimental groups treated with peptide β1-42 (referred to as Beta), peptide β1-42 + *Salvia miltiorrhiza* (referred to as Salvia), Peptide β1-42 + palmitoylethanolamide (referred to as PEA), peptide β1-42 + D-aspartate (referred to as Aspartate), peptide β1-42 + (*salvia miltiorrhiza +* palmitoylethanolamide) (referred to as Salvia + PEA), peptide β1-42 + (*salvia miltiorrhiza +* palmitoylethanolamide + D-aspartate) (referred to as Formula). N=5-7 for each experimental group. *P < 0.05 vs old, **P < 0.01 vs *old* (*old*: old object explored in the *training* step; *new*: new object not explored in the *training* stage).

Both when the animals are administered with composition I according to the invention (Salvia + PEA) and when animals are administered with composition II according to the invention (Formula = Salvia + PEA + Aspartate), the increase in time spent by the animals in exploring the new object is significant compared to the exploration of the old object during the testing stage; this means that the animals have retained the memory of the old object already explored during the training stage, and thus shows an improvement in their cognitive state.

Figure 5 shows the results of the Y-maze test, expressed in terms of % of corrected alternations, at 3 weeks (1 week of settling + 3 weeks of testing) in the untreated experimental group (control group, CRTL) and in the experimental groups treated with β1-42 peptide (Beta), peptide β1-42 *+ salvia miltiorrhiza* (Salvia), peptide β1-42 + palmitoylethanolamide (PEA), peptide β1-42 + D-aspartate (Aspartate), peptide β1-42 + (*salvia miltiorrhiza +* palmitoylethanolamide) (Salvia + PEA), peptide β1-42 + (*salvia miltiorrhiza +* palmitoylethanolamide + D-aspartate) (Formula). N=5-7 for each experimental group. *P < 0.05 vs Beta; **P <0.01 vs CTRL.

Figure 5 shows that the experimental group of mice treated with composition II according to the invention (Formula = Salvia + PEA + Aspartate) has a higher percentage of correct alternations than the other experimental groups, with a significance of *P < 0.05 vs the experimental group treated with peptide β1-42 (Beta). Therefore, as can be seen from these experimental data, mice treated with composition II according to the invention (Formula = Salvia + PEA + Aspartate) show an improvement in cognitive functions compared to mice treated with single ingredients or with a combination thereof.

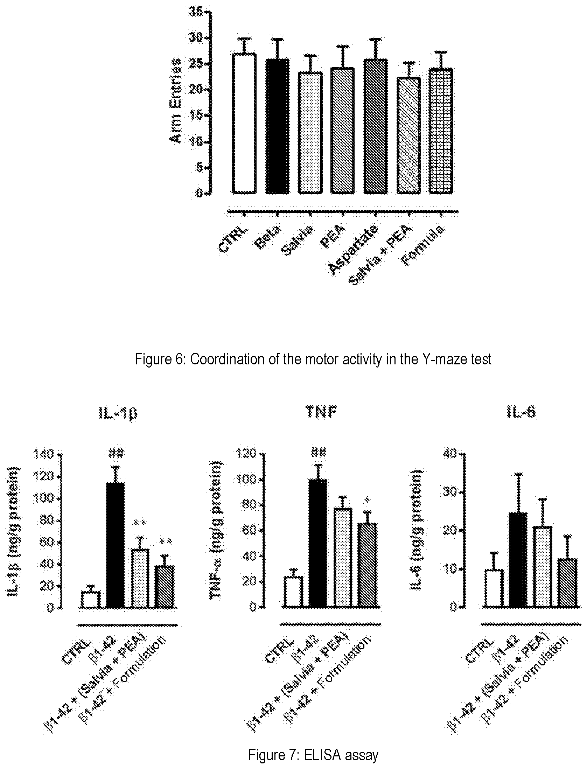

In none of the experimental conditions was an alteration in motor coordination detected by the Y-maze test (Figure 6).

Figure 7 shows the levels of the pro-inflammatory cytokines I L-1 β (A), TNFa (B) and IL-6 (C) determined by ELISA and expressed as ng per mouse (ng/mouse) in mouse hippocampus and cortex lysates (ex vivo biochemical assays) treated with saline (CTRL), peptide β1-42, peptide β1-42 + (*salvia miltiorrhiza +* palmitoylethanolamide) (Salvia + PEA) and finally peptide β1-42 + (*salvia miltiorrhiza +* palmitoylethanolamide + D-aspartate) (Formula). N=5-7 for each experimental group. ## P < 0.01 vs CTRL; *P < 0.05, **P < 0.01 vs β1-42.

Both when animals are administered with composition I according to the invention (Salvia + PEA) and when animals are administered with composition II according to the invention (Formula = Salvia + PEA + Aspartate), the reduction of pro-inflammatory cytokine levels compared to animals treated with only β1 -42 (Beta) peptide (model generation of Alzheimer's) is significant.

## Claims

1. A composition comprising an effective amount of a mixture that comprises or alternatively consists of:
a) palmitoylethanolamide,
b) extract of *Salvia miltiorrhiza* with a titre of IIA tanshinones of 10% by weight/total weight of the dry extract, and
c) D-aspartate
for use in a preventive and/or curative method of treatment of cognitive deficits induced by neurodegenerative pathologies.

2. The composition for use according to any one of the preceding claims, wherein a) it is present in an amount by weight of from 10 mg to 5000 mg, b) it is present in an amount by weight of from 10 mg to 10000 mg and/or c) it is present in an amount by weight ranging from 10 mg to 10000 mg.

3. The composition for use according to any one of the preceding claims, wherein said composition for use is administered orally.

4. A pharmaceutical composition or food supplement comprising an effective amount of a mixture which comprises or, alternatively, consists of:
a) palmitoylethanolamide,
b) extract of *Salvia miltiorrhiza* with a titre of IIA tanshinones of 10% by weight/total weight of the dry extract, and
c) D-aspartate
and, optionally, at least one inert ingredient, additive or excipient acceptable for pharmaceutical, nutraceutical or food use.

## Patentansprüche

1. Zusammensetzung, umfassend eine wirksame Menge einer Mischung, die Folgendes umfasst oder alternativ aus Folgendem besteht:
a) Palmitoylethanolamid,
b) Extrakt von *Salvia miltiorrhiza* mit einem Titer von IIA-Tanshinonen von 10 Gew.-%/Gesamtgewicht des Trockenextrakts und
c) D-Aspartat
zur Verwendung bei einem vorbeugenden und/oder kurativen Verfahren zur Behandlung von kognitiven Defiziten, die durch neurodegenerative Pathologien induziert sind.

2. Zusammensetzung zur Verwendung nach einem der vorhergehenden Ansprüche, wobei a) sie in einer Gewichtsmenge von 10 mg bis 5000 mg vorhanden ist, b) sie in einer Gewichtsmenge von 10 mg bis 10000 mg vorhanden ist und/oder c) sie in einer Gewichtsmenge im Bereich von 10 mg bis 10000 mg vorhanden ist.

3. Zusammensetzung zur Verwendung nach einem der vorhergehenden Ansprüche, wobei die Zusammensetzung zur Verwendung oral verabreicht wird.

4. Pharmazeutische Zusammensetzung oder Nahrungsergänzungsmittel, umfassend eine wirksame Menge einer Mischung, die Folgendes umfasst oder alternativ aus Folgendem besteht:
a) Palmitoylethanolamid,
b) Extrakt von *Salvia miltiorrhiza* mit einem Titer von IIA-Tanshinonen von 10 Gew.-%/Gesamtgewicht des Trockenextrakts und
c) D-Aspartat
und optional mindestens einem inerten Bestandteil, Zusatzstoff oder Hilfsstoff, der für eine pharmazeutische, nutrazeutische oder Nahrungsmittelanwendung unbedenklich ist.

## Revendications

1. Composition comprenant une quantité efficace d'un mélange qui comprend ou qui est constitué, alternativement, de :
a) palmitoyléthanolamide,
b) un extrait de *Salvia miltiorrhiza* présentant un titre en tanshinone IIA de 10 % en poids/poids total de l'extrait sec, et
c) D-aspartate
destinée à être utilisée dans un procédé préventif et/ou curatif de traitement de déficits cognitifs induits par des pathologies neurodégénératives.

2. Composition destinée à être utilisée selon l'une quelconque des revendications précédentes, dans laquelle a) est présent en une quantité en poids de 10 mg à 5 000 mg, b) est présent en une quantité en poids de 10 mg à 10 000 mg et/ou c) est présent en une quantité en poids allant de 10 mg à 10 000 mg.

3. Composition destinée à être utilisée selon l'une quelconque des revendications précédentes, ladite composition destinée à être utilisée étant administrée par voie orale.

4. Composition pharmaceutique ou complément alimentaire comprenant une quantité efficace d'un mélange qui comprend ou qui est constitué, alternativement, de :
a) palmitoyléthanolamide,
b) un extrait de *Salvia miltiorrhiza* présentant un titre en tanshinone IIA de 10 % en poids/poids total de l'extrait sec, et
c) D-aspartate
et, éventuellement, au moins un ingrédient inerte, additif ou excipient acceptable pour un usage pharmaceutique, nutraceutique ou alimentaire.
